# EUROPEAN PATENT SPECIFICATION

(11) **EP 1 675 635 B1**
(45) Date of publication and mention of the grant of the patent: **05.08.2009**
(21) Application number: 04749001.6
(22) Date of filing: 15.06.2004
(51) Int. Cl.: A61M 15/00, A61K 9/14, A61J 3/02

(54) **a multi-substance dry powder inhaler device**
trockenpulverinhalatorvorrichtung für mehrstoffgemische
inhalateur de poudre seche pour melanges complexes

(30) Priority: 19.06.2003 SE 0301815
(43) Date of publication of application: 05.07.2006
(73) Proprietor: Mederio AG, 6052 Hergiswil NW (CH)
(72) Inventor: NILSSON, Thomas, S-647 32 Mariefred (SE)
(74) Representative: Stenborg, Anders Vilhelm
(86) International application number: PCT/SE2004/000952
(87) International publication number: WO 2004/110539

(56) References cited:
- EP-A1- 0 469 814
- WO-A-01/17595
- WO-A-01/68169
- WO-A-03/035137
- WO-A1-00/64519
- WO-A1-01/39823
- WO-A1-03/061743
- GB-A- 2 242 134

## Description

### TECHNICAL FIELD

The present invention relates to a dry powder inhaler device designed to administer medicaments by an oral inhalation, comprising at least two medicinal dry powders., More particularly, the invention relates to a dry powder inhaler device for simultaneous delivery in a single inhalation of separate dry powder formulations of different medicaments constituting a combined dose.

### BACKGROUND

Administration of drugs by an oral inhalation route is very much in focus today, because of advantages offered like rapid and predictable onset of action, cost effectiveness and high level of comfort for the user. There are mainly two types of inhalers on the market, pressurized metered dose inhalers (pMDIs) comprising a suspension of fine medicament particles in a propellant gas and dry powder inhalers (DPIs) comprising fine medicament particles as dry powder.

Dry powder inhalers (DPI) attract perhaps the most interest, compared to pMDIs, because of the flexibility they offer in terms of nominal dose range i.e. the amount of active substance that can be administered in a single inhalation. So far most development efforts have been directed towards producing effective drugs and formulations for specific abnormal conditions and not so much towards developing the delivery device, i.e. the inhaler.

When inhaling a dose of dry medication powder it is important to obtain by mass a high fine particle fraction (FPF) of particles with an aerodynamic size preferably less than 5 µm in the inspiration air. The majority of larger particles does not follow the stream of air into the many bifurcations of the airways, but get stuck in the throat and upper airways. It is not uncommon for prior art inhalers to have an efficacy of 10 - 20 % only, i.e. only 10 - 20 % of the metered dose by mass is actually delivered as particles with an aerodynamic size less than 5 µm. Since most drugs have undesirable side effects and some may be quite toxic if overdosed, it is important to keep the dosing to the user as exact as possible and to design the delivery system, e.g. an inhaler, such that the efficacy becomes much higher than 10 - 20 %, thereby reducing the required amount of drug in the dose. Also, depending on the targeted site of action, be it systemic or local in the throat and airways, it is desirable to tailor the physical formulation of a medication powder in such a way that it results in an advantageous particle aerodynamic size distribution by mass in the metered dose.

An interesting field of research concerns the possibility of simultaneous administration of combinations of different medicaments. Of course, it is well known in prior art that a successful treatment of a medical condition may require administration of more than one active substance, e.g. a medicament for relaxing the immediate symptoms like pain or bronchoconstriction and another medicament for treating the underlying abnormal condition like systemic chemical imbalance or airway inflammation respectively. However, it is often difficult to mix the medicaments into a metered dose, because the medicaments may be known to be incompatible or else it is perhaps unknown if and how they interact before they are actually delivered to a subject. Therefore, medicaments are generally administered separately, in sequence or by separate routes.

In the past decade research into respiratory diseases, their prophylaxis and treatment, has shown conclusively that simultaneous administration of combinations of different medicaments may improve the clinical condition of patients considerably. In Switzerland patients diagnosed with asthma have been prescribed FORADIL (formoterol, a bronchodilating substance) together with PULMICORT (budesonide, an anti-inflammatory steroid) since the 1980's for treatment of their asthma. Until recently, however, different asthma medicaments have generally been administered separately, in sequence or by separate routes, not in compositions comprising more than one active ingredient. However, there are several published patent applications and approved patents teaching methods of treating respiratory disorders like asthma and chronic obstructive pulmonary disease (COPD) and pharmacologic compositions of different biologic and chemical substances for this purpose, where the combinations offer overall advantages in the treatment of these conditions. See for instance EP 0416950B "Medicaments", EP 0416951B1 "Medicaments comprising salmeterol and fluticasone", EP 0613371B1 "New combination of formoterol and budesonid", WO 98/15280 "New combination", WO 00/48587 "Combinations of formoterol and fluticasone propionate for asthma", WO 01/70198A1 "Stabilized dry powder formulations", WO 01/78737A1 "Medical combinations comprising formoterol and budesonid", WO 01/78745A1 "Medical combinations comprising formoterol and fluticasone propionate", WO 02/28368A1 "New combination for the treatment of asthma", WO 03/013547A1 "Pharmaceutical composition comprising salmeterol and budesonid for the treatment of respiratory disorders". However, the quoted documents deal with aspects of formulating, processing, stabilizing and using mixtures of at least two ingredients. The chemical compositions and mixing ratios between active ingredients are generally focused upon, not methods of administration of such compostions or devices for that purpose. It is, however, difficult to mix dry medicament powders and optional excipients in a certain proportion consistently. The proportions in such a metered combined dose cannot be easily controlled, because the ratio of medicaments in an individual, combined dose depends significantly on the particle forces existing in each medicament powder, between particles of different medicaments and between medicament powders and dose packaging materials. Hence, actual variations in the ratio between active ingredients from combined dose to combined dose may be too large, causing serious problems if a potent ingredient is delivered in a higher or lower amount than expected.

WO 01/117595 discloses an inhaler comprising a rotatable metering member for dispensing a measured amount of material, a delivery passage and a delivery orifice and at least one actuator member adapted to move the metering member from a retaining position to a material dispensing position. The inhaler may be used to deliver a single or a combination therapy.

Thus, there is room for improvements regarding methods and devices for simultaneous administration of different medicaments, which combine advantageously in treatment of certain medical disorders.

### SUMMARY

A dry powder inhaler devices comprises a nozzle, a common dose bed, and a combined dose of finely divided dry powders intended for delivery by the inhaler device. This combined dose comprises pre-metered quantities of at least two substances separately deposited on the common dose bed. The inhaler device also comprises an arrangement for providing a relative motion between the nozzle and the common dose bed. This relative motion means that the nozzle passes over the combined dose for a simultaneous or sequential delivery of the substances deposited thereon during the course of a single suction of air.

The deposits of the at least two medicaments are suitably kept separated from each other, such that the medicaments cannot detrimentally interact after forming of the combined dose, and the medicinal combined dose can be introduced into an inhaler device for a delivery of the medicinal combined dose during the course of a single inhalation, whereby the delivered medicinal combined dose is composed to a high degree by mass of de-aggregated fine particles of each of the at least two medicaments.

Furthermore a therapeutic metered medicinal, combined dosage of finely divided dry medication powders is disclosed wherein the therapeutic, metered combined dosage comprises metered quantities of at least two medicaments, separately deposited; and the medicinal combined dosage is adapted for a user initiated delivery of the combined dosage during the course of a single inhalation through an inhaler device. The at least two medicaments of the medicinal combined dosage are aerosolized generally simultaneously or generally sequentially during an inhalation, depending on how the dosage is physically composed, whereby a delivered dosage to a user consists of a high degree by mass of fine particles of each medicament such that a large proportion of each medicament settles in the intended target area in the airways and lungs of the user.

### BRIEF DESCRIPTION OF THE DRAWINGS

The invention, together with further objects and advantages thereof, may best be understood by referring to the following detailed description taken together with the accompanying drawings, in which:
- FIG. 1: illustrates in top and side views a first embodiment of combined doses comprising two medicament deposits in separate compartments onto a dose bed;
- FIG. 2: illustrates in top and side views a second embodiment of combined doses comprising three medicament deposits in separate compartments onto a dose bed;
- FIG. 3: illustrates in top and side views a third embodiment of combined doses comprising two parallel medicament deposits onto a dose bed;
- FIG. 4: illustrates in top and side views a fourth embodiment of combined doses comprising several medicament deposits and separating excipient deposits onto a dose bed;
- FIG. 5: illustrates in top and side views a fifth embodiment of combined doses comprising four medicament deposits and separating excipient deposits onto a dose bed;
- FIG. 6: illustrates in top and side views a sixth embodiment of combined doses comprising two parallel medicament deposits on top of one another onto a dose bed;
- FIG. 7: illustrates in top and side views a seventh embodiment of combined doses comprising two medicament deposits on top of one another onto a dose bed, but separated by a deposit of an excipient;
- FIG. 8: illustrates in top and side views another embodiment of a combined dose comprising two medicaments separately deposited onto a dose bed;
- FIG. 9: illustrates in top and side views yet another embodiment of a combined dose comprising two medicaments separately deposited onto a dose bed, but with some degree of overlap;
- FIG. 10a: illustrates in a sectional view an example of a combined dose comprising two medicament deposits on top of one another but separated by a deposit of an excipient onto a dose bed and adjacent to the combined dose a nozzle in a starting position before the combined dose is released; and
- FIG. 10b: illustrates in a sectional view an example of a combined dose comprising two medicament deposits on top of one another but separated by a deposit of an excipient onto a dose bed and adjacent to the combined dose a nozzle in a relative motion sucking up the powder particles to be dispersed into the air stream.

### DETAILED DESCRIPTION

The present invention is based on a new method of forming combined doses comprising more than one medicament deposited onto a dose bed and a new dry powder inhaler (DPI) for delivering such combined doses by an inhalation route to a user of the DPI .

In the context of this application the word "medicament" is defined as a pharmacological substance, which comprises at least one chemically or biologically active agent. Further, a medicament may exist in a pure form of one or more pure active agents, or a medicament may be a compound comprising one or more active agents, optionally formulated together with other substances, e.g. enhancers, carriers, diluents or exipients. From this point on, the term "excipient" is used to describe any chemical or biologic substance mixed in with a pure active agent for whatever purpose. In this document, only medicaments in dry powder form are discussed.

A "dose bed" is henceforth defined as a member capable of harboring a metered dose of one or more dry powders, where the dose is intended for a delivery to a user of a DPI in a single inhalation performed by the user. In the present invention a combined dose comprises metered deposits of at least two medicaments. The dose bed may be divided in several areas or incorporate two or more compartments intended for deposits of dry powders. In a preferred embodiment the combined dose is packaged for a continuous, prolonged delivery, i.e. the delivery period is in a range 0.01 to 6 s, usually in a range 0.1 to 2 seconds, delivery taking place sometime during the course of an inhalation as controlled by a purposefully designed DPI. Advantageously, such a DPI adopts an Air-razor method of gradual aerosolization of the combined dose comprising introduction of a relative motion between an air-sucking nozzle and the powder dose. Advantages of a prolonged delivery of a dose for inhalation are disclosed in our US Patent No. 6,571,793 (WO 02/24264 A1).

A preferred embodiment of a metered combined dose uses a dose bed split up in at least two separate compartments, where each compartment is intended for a metered deposition of a particular medicament. Each compartment containing a metered amount of a specified medicament powder may then be sealed, e.g. by foiling, such that the different medicaments in the different compartments of the dose bed cannot interact in any way and can not be contaminated by foreign substances or moisture. Alternatively, a common foil encloses all compartments, but sealing between compartments may be excluded if individual sealing is not a requirement. A dose carrier is normally engaged to carry at least one dose bed loaded with a dose, whereby the dose carrier may be inserted into a DPI for administering a combined dose or doses sequentially to a user in need of treatment. A suitable carrier of doses is disclosed in our Swedish patent publication SE 0517 806 C2 (WO01/34233 A1). However, a dose bed may be designed to act as a carrier, intended for direct insertion into a DPI. A suitable DPI for a continuous dose delivery is disclosed in our US patent No. 6,422,236 B1.

If complete physical separation of the deposits of the different medicaments making up the combined dose is not required, but some degree of overlap or mixing is acceptable from a physical, chemical and medical point of view, then other methods of separating the deposits may be implemented. Depending on what degree of mixing is permitted different ways of separating deposits must be adopted. For example, in one embodiment, the different medicaments may be deposited in parallel strings onto the dose bed. The dose bed may use separate indentations where the powder should be deposited, but flat target areas for deposits in a single plane on the dose bed are equally possible. In another embodiment the different medicaments are deposited sequentially dot-wise or string-wise onto different target areas of the dose bed. Yet another way of depositing the medicaments would be on top of one another, in layer by layer, such that each medicament is deposited on top of the previously deposited one. If necessary, to stop chemical or biological interaction or decomposition caused by, for example, adjacent medicament powders being incompatible, an isolating layer of a biologically acceptable, inert substance like carbohydrates, e.g. glucose or lactose, may be deposited between adjacent layers of medicament. A similar method of separation may also be used to positively separate adjacent dots or strings of medicaments, by depositing an inert substance between adjacent dots or strings of different medicament deposits onto the dose bed. When the combined dose has been completely formed it is usually sealed from ingress of dirt and moisture by a foil covering the entire dose bed. A method of depositing microgram and milligram quantities of dry powders using electric field technology is disclosed in our US Patent Application No. 2003/0012865 A1.

Forming a combined dose comprising at least two medicaments in separate dry powder formulations may be done in different ways, known in prior art. The invention discloses that the components of the combined dose, i.e. the at least two medicaments need not be mixed or processed together prior to dose forming and, indeed, should normally be kept separated during dose forming as well as after the combined dose is formed and sealed. The medicaments of the combined dose are thus kept separated on the dose bed by a suitable method, as described in the foregoing, until the combined dose is about to be delivered by an inhalation route to a user.

Methods of dose forming include conventional mass or volumetric metering and devices and machine equipment well known to the pharmaceutical industry for filling blister packs, for example. See European Patent No. EP 0 319 131 B1 and US Patent No. 5,187,921, for examples of prior art in volumetric and/or mass methods and devices for producing doses of medicaments in powder form. Electrostatic forming methods may also be used, for example as disclosed in US Patent No. 6,007,630 and US Patent No. 5,699,649. Any method capable of producing metered micro- and milligram quantities of dry powder medicaments may be used, even completely different methods may be applied to suit the different medicaments selected to be part of the combined doses to be produced. Total mass in a combined dose according to the present invention is typically in a range from 50 µg to 50 mg. Regardless of which forming and filling method is being used for a particular medicament, it is important during dose forming to make sure that intended medicaments are individually metered and deposited onto their respective target areas or compartments of the dose bed. The target areas or compartments of the dose bed, which combine to hold a dose, may be of same or differing sizes. The shape of compartments is governed by physical constraints defined by the type of dose bed used. As an example, a preferred type of dose bed is an elongated strip of a biologically acceptable, inert material, e.g. plastic or metal, between 5 and 50 mm long and between 2 and 10 mm wide. The strip is further divided in separate target areas or compartments arranged along the length of the elongated strip. The dose bed or, if necessary each compartment, receives an individual seal, for instance in the form of a foil, in a step immediately subsequent to the dose forming.

An advantage of the present invention is that a potentially interesting medicament may be individually selected on merits of its own for inclusion in a combined dose, in disregard of whether or not it is chemically or biologically compatible with other potentially interesting medicaments. The combined dose may be designed to include medicaments, which have proven medical effects of different, desirable kinds, even though the selected medicaments may be chemically or biologically incompatible or unstable in the form of a mixture. Thus, the regulatory process before introducing combined doses of medicament combinations on the market may be drastically simplified. Yet another advantage of the invention is the possibility of using pure, more or less potent medication substances as selected medicaments of the combined dose, without included excipients. Non-exclusive, illustrative examples not limiting the scope of the invention of suitable typical medicaments for treatment of asthma and COPD to be combined in single combined doses in accordance with the present invention are listed below:
Formoterol and Budesonide
Formoterol and Ipratropium
Formoterol and Fluticasone
Formoterol and Tiotropium
Ipratropium and Budesonide
Ipratropium and Salbutamol

Illustrative examples, not limiting the scope of the invention and analogous with respiratory medicaments of suitable medicaments for pain control, which may be advantageously combined to be combined in single combined doses according to the present invention, include non-exclusively:
Almotriptan
Analgesics
Anticonvulsants
Antidepressants
Antiemetics
Aspirin (lysine acetylsalicylic acid)
Betablockers
Calcium channel antagonists
Codeine
DHE
Domperidone
Eletriptan
Ergotamine
Frovatriptan
Metoclopramide
Naratriptan
Isometheptene
Opiates
Paracetamol
Rizatriptan
Serotonin
Sumatriptan
Triptans
Zolmitriptan

Optimal dosages of the respective active substances for prevention or treatment of disorders may be determined by those skilled in the art, and will vary with the type of disorder, selected compounds and their respective potency, and the advancement of the disease condition. Furthermore, factors associated with the individual undergoing treatment determine correct dosages, such as age, weight, sex etc. Depending on what are correct dosages, the correct deposits by mass for the prepared medicaments may be calculated, such that metered deposits of each medicament to be included in the metered combined dose may be produced in a dose-forming step. In calculating a correct nominal deposit of mass for each medicament component the fine particle fraction, i.e. particles having a mass median aerodynamic diameter (MMAD) less than 5 µm, per component of the actual delivered dose must be taken into consideration. As discussed in the foregoing, the efficacy of inhalers differs considerably and it is thus important to include the expected efficacy of the chosen inhaler in the calculation of what is a suitable nominal mass deposit. Another parameter to consider when forming the combined dose is the physical formulation of included medication powders. Formulation objectives may differ for the different medicament components of the combined dose. The particle aerodynamic size distribution by mass may be targeted differently for the different dose components in order to optimize the efficacy of each component in the treatment of a particular disease in a host user. For instance, the MMAD for a steroidal medicament component should be larger than that of a bronchodilating medicament component. Whereas maximum penetration into the lungs is required of a bronchodilator, a minimum of systemic absorbance and maximum local deposition in the targeted area of the airways is required of the steroid.

Compared to prior art, more opportunities are opened up by the present invention in selecting medicaments based on existing compositions with proven medical effects, rather than first developing a mixture or formulation of different medicaments and then proving that the new combination is effective, stable and lacks serious side effects. The present invention makes it possible to define combined doses using any combination of pure medicaments, i.e. pure pharmacologic agents, and medicaments comprising excipients. A combined dose thus formed onto a dose bed may be introduced into a dry powder inhaler (DPI) such that the medicament components making up the combined dose may be aerosolized and delivered in the inspiration air during the course of an inhalation through the DPI by a user.

The invention also offers interesting opportunities for combinations of new medicaments and combinations of new medicaments with existing, proven ones. Keeping the different medicaments separated according to the invention may reduce the investment in time and resource necessary for getting the combined medicaments approved by the relevant regulatory bodies and released to the respective markets. For instance, no added substance to stabilize the combined product will be needed and no testing to prove that the added substance is harmless needs be performed. New areas of therapy are thus now suitable for treatment by inhalation. Besides asthma, COPD and pain, other examples not limiting the scope of the invention, of medical areas of therapy, where combinations of medicaments administered in single combined doses by an inhalation route may improve the quality of treatment, lower the costs and make life for patients more comfortable, are non-exclusively:
Disorders of the alimentary tract or the digestive system
Disorders of the cardiovascular system
Disorders of the endocrine system
Disorders of the respiratory system
Genital or sexual disorders
Disorders of the muscular or neuromuscular system
Disorders of the nervous system
Psychosomatic disorders
Infection restrainers
Allergic disorders
Protective or antinoxious agents

The present invention differs from prior art inhalers and related dose delivery methods by providing a combined dose comprising two or more separate medicaments, more or less separately deposited onto a dose bed. The combined dose is therefore not a composition of medicaments constituting a single physical entity, but rather two or more physical entities contained in a single dose. Inserted into a DPI, the combined dose will be aerosolized such that the entities of the dose, i.e the medicaments, are delivered mostly sequentially or optionally mostly simultaneously into the inspiration air during an inhalation by a user. Whether medicaments included in a combined dose are aerosolized mainly sequentially or mainly simultaneously depends partly on the physical form of the combined dose, i.e. how the medicament deposits are interrelated and partly on what type of inhaler is used to administer the combined dose. It is obvious that an inhaler, which subjects all of the combined dose to a jet-stream of air will aerosolize the included deposits simultaneously and more or less mixed, whereas an inhaler subjecting the combined dose to a jet stream gradually, like a moving tornado, thereby not attacking all of the combined dose at once, may aerosolize the deposits of the dose gradually over time. An object of the invention is to offer better control of combined dose release and to facilitate a prolonging of the dose delivery in order to produce a high fine particle fraction (FPF) in the delivered, combined dose. Another object of the invention is to achieve a high ratio of delivered, combined dose relative metered combined dose. Although it is possible to successfully apply the invention to prior art inhalers, these tend to deliver the dose in too short a time, resulting in a poor FPF figure and low efficacy. On the other hand, a gradual dose delivery is possible using a new inhaler design where a relative movement is introduced between the dose and a suction nozzle through which the inspiration airflow is channeled. This arrangement utilizes the inhalation effort of the user to aerosolize the combined dose gradually for a prolonged period, thus using the power of the suction more efficiently and eliminating in most cases a need for external power to aerosolize the combined dose.

A powder Air-razor method is advantageously used for aerosolizing the medicament powders in the combined dose, the Air-razor providing de-aggregation and dispersal into air of the finely divided medication powders. Utilizing an effort of sucking air through a mouthpiece of an inhaler, said mouthpiece connected to a nozzle, the particles of the deposited medicament powders, made available to the nozzle, are gradually de-aggregated and dispersed into a stream of air entering the nozzle. The gradual de-aggregation and dispersal is produced by the high shearing forces of the streaming air and a relative motion introduced between the nozzle and the powders of the combined dose. In a preferred embodiment, the medicament powders are deposited onto a dose bed, such that the powder deposits occupy a larger area than the area of the nozzle inlet. The nozzle is preferably positioned outside the deposited area, not accessing the powder by the relative motion until the air stream into the nozzle, created by the suction, has passed a threshold flow velocity. Coincidental with the application of the suction or shortly afterwards the relative motion will begin such that the nozzle traverses the powder dose gradually. The high velocity air going into the nozzle inlet provides plenty of shearing stress and inertia energy as the flowing air hits the leading point of the border of the contour of the first medicament deposit. This powder Air-razor method, created by the shearing stress and inertia of the air stream, is so powerful that the particles in the particle aggregates in the powder adjacent to the inlet of the moving nozzle are released, de-aggregated to a very high degree as well as dispersed and subsequently entrained in the created air stream going through the nozzle. If the medicament deposits have been made in separate compartments of the dose bed and individually sealed, then obviously the compartments must be opened up first so that the nozzle can access the deposited powder in each compartment when suction is applied. Naturally, this is also true if the deposits share a common seal without an individual seal for each deposit. An arrangement for this purpose is disclosed in our Swedish patent publication SE 517 227 C2 (WO 02/24266 A1).

Depending on how the deposits are laid out on the dose bed, the nozzle will either suck up the deposits sequentially or in parallel or in some serial/parallel combination.

Thus, the quality of combined dose delivery is dramatically improved compared to prior art performance, and leading to important advances in delivering a majority of fine particles of the medicaments of the combined dose to the intended target area or areas in the user's airways and lungs with very little loss of particles settling in the throat and upper airways. Administering medicament combinations according to the present invention has a very positive therapeutic effect from a medical, psychological and social point of view on a host in need of treatment with a combination of at least two medicaments.

### Detailed descriptions of drawings

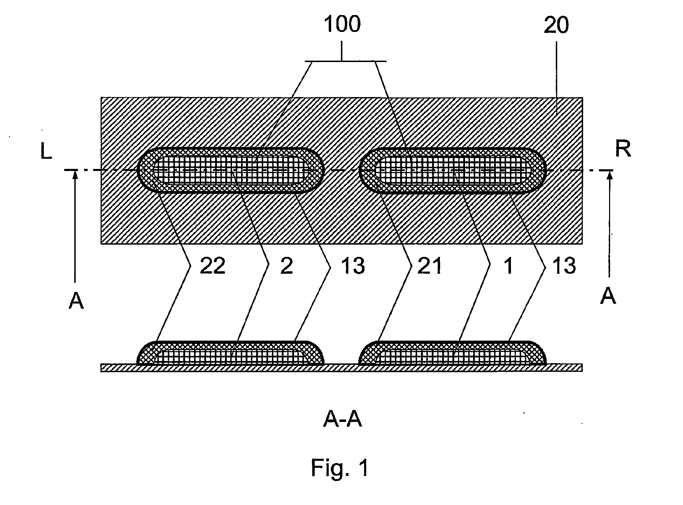

Referring to reference numbers 1 - 100 of the drawings wherein like numbers indicate like elements throughout the several views of ten different embodiments of a combined dose comprising at least two deposits of at least two medicaments onto a dose bed as illustrated in Figures 1 - 10 presented here as non-limiting examples.

Figure 1 illustrates a combined dose **100** comprising two different medicament deposits, **1** and **2,** in separate compartments **21** and **22** onto a dose bed **20,** which may be capsules or blisters or moldings in the dose bed. An individual seal **13** for each compartment guarantees that the medicaments cannot be contaminated by foreign matter or by one another. The illustrated deposits are intended for a sequential delivery taking place during an inhalation.

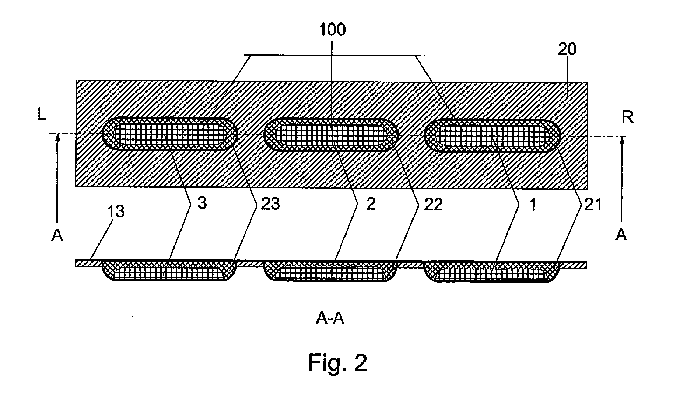

Figure 2 illustrates a combined dose **100** comprising three different medicament deposits, **1, 2** and **3** in separate compartments **21, 22** and **23** similar to Figure 1, but arranged underneath the dose bed **20.** Besides a different arrangement of compartments on the dose bed **20** and the respective seals **13,** the main difference between Figure 1 and Figure 2 is that deposit **3** may consist of a different medicament from deposits **1** and **2** or it may consist of either the medicament of deposit **1** or **2.** It is thus possible not only to administer more than one medicament, but also to compose combined doses of e.g. two medicaments with a very high ratio of mass between them. The illustrated deposits are intended for a sequential delivery taking place during an inhalation.

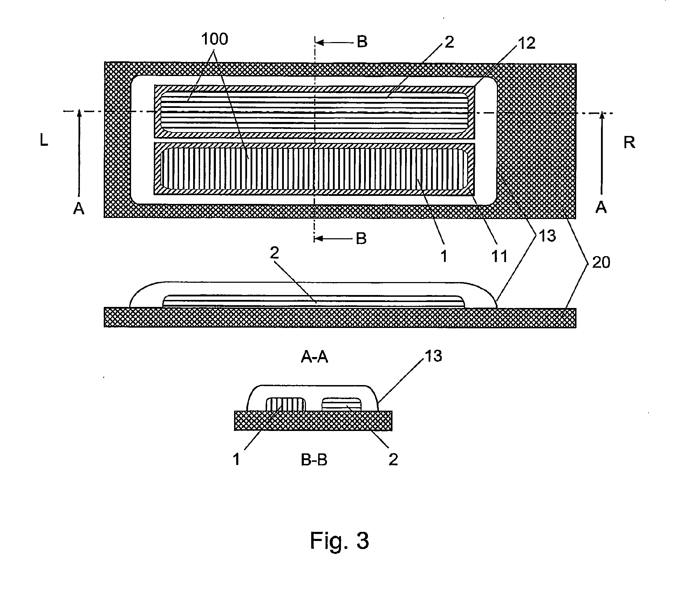

Figure 3 illustrates a combined dose **100** comprising two different medicament deposits, **1** and **2,** laid out in parallel strips onto separate target areas **11** and **12** respectively onto the dose bed **20.** A common protective foil **13** protects the medicaments of the combined dose from being contaminated by foreign matters. The illustrated deposits are intended for a fully simultaneous delivery of the two medicaments taking place during an inhalation.

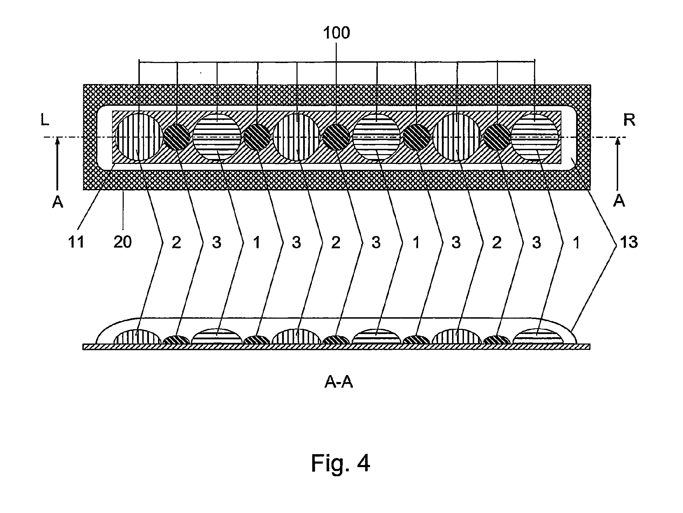

Figure 4 illustrates a combined dose **100** comprising two different medicaments, **1** and **2,** each comprising several deposits separated by deposits of an inert excipient **3.** The deposits are laid out in a string of spots onto a target area **11** on a dose bed **20.** The deposits share a common seal **13.** The combined dose is intended for a sequential delivery of incorporated medicament spots, said delivery taking place during an inhalation. The excipient deposits help to minimize unintentional mixing of the medicaments. If some mixing of medicaments can be accepted, then the excipient may be left out altogether. Combined doses composed of spot deposits may of course comprise more medicaments than two. The mass ratio between medicaments may be easily set by controlling the ratio between the number of spots per medicament in combination with the size of the respective spots in terms of deposited mass. Naturally the spots need not necessarily be circular in shape, they may take an elongated or elliptical form, depending on which type of dose forming method is used.

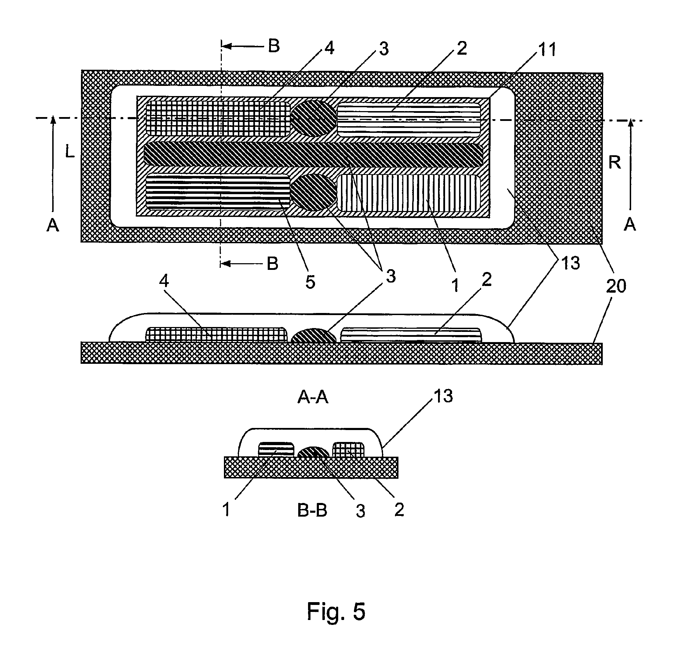

Figure 5 illustrates a combined dose **100** comprising deposits of four different medicaments, **1, 2, 4** and **5,** separated by deposits of an inert excipient **3.** The deposits are laid out in two parallel groups of two in-line strips of medicament onto a common target area **11** on a dose bed **20.** The deposits share a common seal The excipient deposits help to minimize unintentional interaction of the medicaments. The combined dose is intended for a combined parallel/simultaneous and sequential delivery of incorporated medicament strips, said delivery taking place during an inhalation.

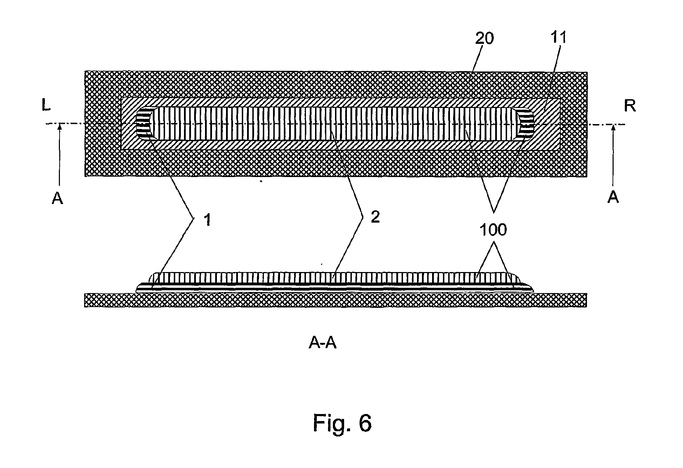

Figure 6 illustrates a combined dose **100** comprising two different medicaments, **1** and **2,** each comprising a strip of deposited powder, medicament **1** deposited onto a target area **11** of a dose bed **20** and medicament **2** deposited on top of the deposit of medicament **1.** This method of dose forming is an alternative to the ones previously disclosed and may be used when a certain level of interaction of the medicaments can be tolerated.

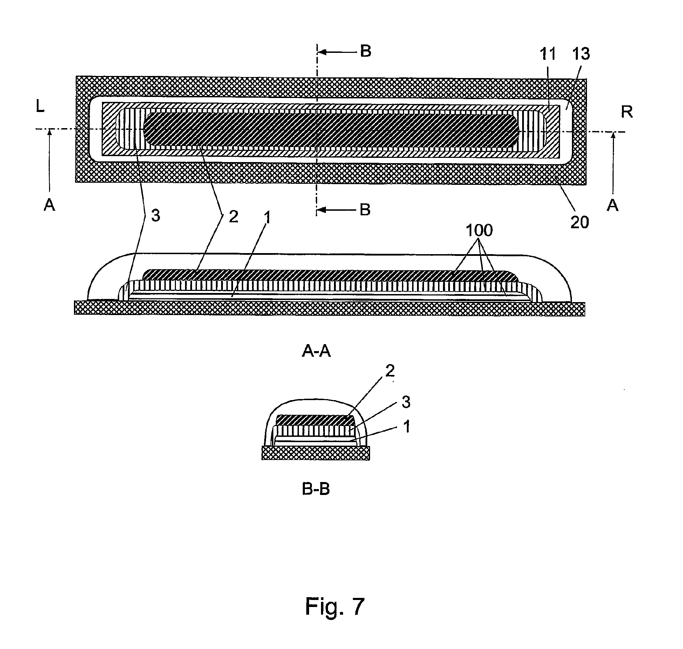

Figure 7 illustrates a combined dose **100** comprising two different medicaments, **1** and **2,** and an excipient **3,** each comprising a strip of deposited powder. Medicament **1** is deposited onto a target area **11** of a dose bed **20** and excipient **3** is deposited onto medicament **2** to insulate medicament **1** from a deposit of medicament **2** on top of the deposits of medicament **1** and excipient **3.** This way of forming doses is not restricted to include only two medicaments, but several medicaments may be deposited on top of one another, if necessary with an insulating deposit of excipient between layers.

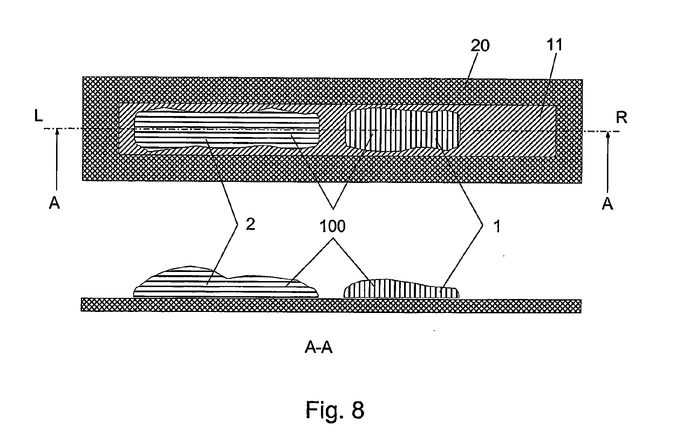

Figure 8 illustrates a combined dose **100** comprising two different medicament deposits, **1** and **2,** of somewhat irregular shapes but separately laid out onto a common target area **11** of the dose bed **20.** The illustrated deposits are intended for a sequential delivery of the two medicaments taking place during an inhalation.

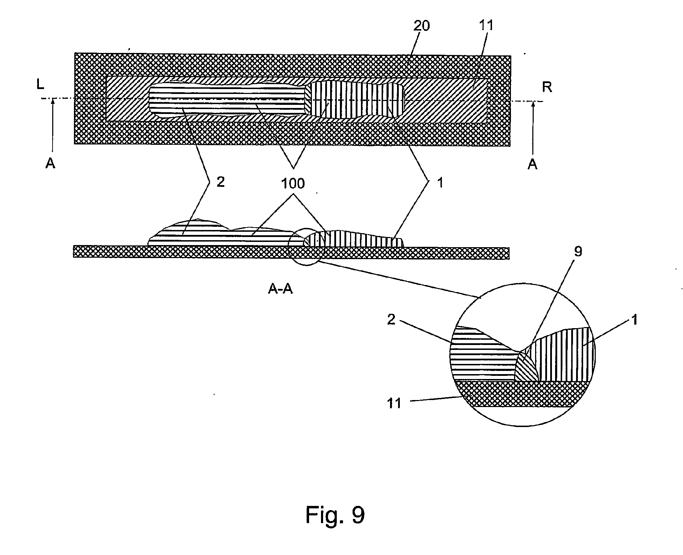

Figure 9 illustrates a combined dose **100** comprising two different medicament deposits, **1** and **2,** of somewhat irregular shapes but generally separately laid out onto a common target area **11** of the dose bed **20.** The illustrated deposits overlap slightly, resulting in a arbitrary mixture **9.** The deposits are intended for a mostly sequential delivery of the two medicaments taking place during an inhalation.

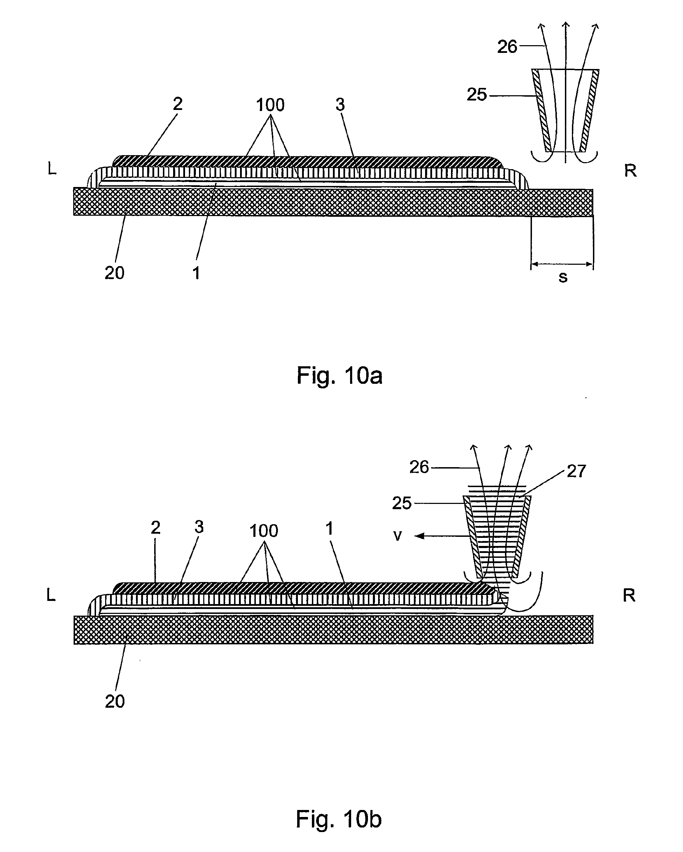

Figure 10a and 10b illustrate a delivery of a combined dose **100** comprising two different medicaments, **1** and **2,** and an excipient **3,** each comprising a strip of powder sequentially deposited in three different layers. A nozzle **25** with an established flow of air **26** going into it is put in a relative motion, parallel to the dose bed **20,** such that the nozzle passes over the combined dose beginning at the right side **R** and ending at the left side **L** of the dose bed. This Air-razor method results in a simultaneous, gradual delivery of medicaments **1** and **2** together with the excipient **3.** The powders of the deposits are mixed into an aerosol **27** by the air flowing into the nozzle leading to simultaneous delivery of the two medicaments and the excipient. This Air-razor method may be applied to all embodiments of the present invention and results in a simultaneous or sequential or a combined simultaneous/sequential delivery of all included medicaments and optional excipients.

## Claims

1. A dry powder inhaler device comprising:
a nozzle (25);
a common dose bed (20); and
a combined dose (100) of finely divided dry powders (1, 2) intended for delivery by the dry powder inhaler device, the combined dose (100) comprises pre-metered quantities of at least two substances (1, 2), separately deposited on the common dose bed (20), **characterized by:**
an arrangement for providing a relative motion between the nozzle (25) and the common dose bed (20), such that the nozzle (25) passes over the combined dose (100) for a simultaneous or sequential delivery of the substances (1, 2) during the course of a single suction of air and the relative motion.

2. The dry powder inhaler device according to claim 1, **characterized by** a mouthpiece connected to the nozzle (25) and adapted for receiving an applied air sucking effort.

3. The dry powder inhaler device according to claim 1 or 2, **characterized in that** the nozzle (25) is initially positioned outside an area (11, 12) of the common dose bed (20) onto which the substances (1, 2) are being deposited.

4. The dry powder inhaler device according to any of the claims 1 to 3, **characterized in that** the arrangement is arranged for providing the relative motion until an air stream (26) induced in the nozzle (25) exceeds a threshold flow velocity.

5. The dry powder inhaler device according to any of the claims 1 to 4, **characterized in that** the common dose bed (20) is sealed with at least one foil (13) and the dry powder inhaler device further comprises an arrangement for opening the seal allowing the nozzle (25) access to the common dose bed (20).

6. The dry powder inhaler device according to claim 5, **characterized in that** the common dose bed (20) comprises at least two compartments (21, 22), the at least two substances (1, 2) are deposited in separate compartments (21, 22) and each compartment (21, 22) is provided with a separate seal (13) to prevent interaction between separately deposited substances (1, 2).

7. The dry powder inhaler device according to any of the claims 1 to 6, **characterized in that** at least one of the at least two substances (1, 2) is composed of a pure, chemical or biologic agent.

8. The dry powder inhaler device according to any of the claims 1 to 7, **characterized in that** at least one of the at least two substances (1, 2) is presented in form of a dry powder compound consisting of an effective amount of a pure, chemical or biologic agent mixed with suitable excipients (3).

9. The dry powder inhaler device according to any of the claims 1 to 8, **characterized in that** the common dose bed (20) is formed as a blister pack, where the common dose bed (20) is designed to accept separate deposits of the substances (1, 2) making up the pre-metered combined dose (100).

10. The dry powder inhaler device according to any of the claims 1 to 9, **characterized in that** a biologically acceptable, inert substance (3) is deposited between the deposits (1, 2) of the at least two substances to prevent the at least two substances from detrimentally interact after forming of the combined dose (100).

## Patentansprüche

1. Trockenpulver-Inhalatorvorrichtung, umfassend:
eine Düse (25);
ein gemeinsames Dosisbett (20); und
eine kombinierte Dosis (100) von fein zerteilten Trockenpulvern (1, 2), die zur Verabreichung durch die Trockenpulver-Inhalatorvorrichtung vorgesehen sind, wobei die kombinierte Dosis (100) vorab abgemessene Mengen von zumindest zwei Substanzen (1, 2) umfasst, die getrennt auf dem gemeinsamen Dosisbett (20) deponiert sind, **gekennzeichnet durch**:
eine Anordnung zum Bereitstellen einer relativen Bewegung zwischen der Düse (25) und dem gemeinsamen Dosisbett (20), so dass die Düse (25) die kombinierte Dosis (100) für eine gleichzeitige oder aufeinanderfolgende Verabreichung der Substanzen (1, 2) während des Verlaufs eines einzelnen Ansaugens von Luft und der relativen Bewegung überfährt.

2. Trockenpulver-Inhalatorvorrichtung nach Anspruch 1, **gekennzeichnet durch** ein Mundstück, das mit der Düse (25) verbunden und ausgelegt ist, eine aufgebrachte Luftansaugleistung aufzunehmen. '

3. Trockenpulver-Inhalatorvorrichtung nach Anspruch 1 oder 2, **dadurch gekennzeichnet, dass** die Düse (25) zu Beginn außerhalb eines Bereichs (11, 12) des gemeinsamen Dosisbetts (20), auf dem die Substanzen (1, 2) deponiert sind, angeordnet ist.

4. Trockenpulver-Inhalatorvorrichtung nach einem der Ansprüche 1 bis 3, **dadurch gekennzeichnet, dass** die Anordnung zum Bereitstellen der relativen Bewegung angeordnet ist, bis ein Luftstrom (26), der in der Düse (25) erzeugt wird, einen Schwellenwert einer Durchflussgeschwindigkeit übersteigt.

5. Trockenpulver-Inhalatorvorrichtung nach einem der Ansprüche 1 bis 4, **dadurch gekennzeichnet, dass** das gemeinsame Dosisbett (20) mit zumindest einer Folie (13) versiegelt ist und die Trockenpulver-Inhalatorvorrichtung ferner eine Anordnung zum Öffnen der Versiegelung umfasst, die der Düse (25) einen Zugriff auf das gemeinsame Dosisbett (20) ermöglicht.

6. Trockenpulver-Inhalatorvorrichtung nach Anspruch 5, **dadurch gekennzeichnet, dass** das gemeinsame Dosisbett (20) zumindest zwei Kammern (21, 22) umfasst, wobei die zumindest zwei Substanzen (1, 2) in getrennten Kammern (21, 22) deponiert sind und jede Kammer (21, 22) mit einer getrennten Versiegelung (13) versehen ist, um eine Wechselwirkung zwischen getrennt deponierten Substanzen (1, 2) zu verhindern.

7. Trockenpulver-Inhalatorvorrichtung nach einem der Ansprüche 1 bis 6, **dadurch gekennzeichnet, dass** zumindest eine der zumindest zwei Substanzen (1, 2) aus einem reinen, chemischen oder biologischen Wirkstoff besteht.

8. Trockenpulver-Inhalatorvorrichtung nach einem der Ansprüche 1 bis 7, **dadurch gekennzeichnet, dass** zumindest eine der zumindest zwei Substanzen (1, 2) in der Form einer Trockenpulververbindung vorliegt, die aus einer wirksamen Menge eines reinen, chemischen oder biologischen Wirkstoffs besteht, der mit geeigneten Trägerstoffen (3) gemischt ist.

9. Trockenpulver-Inhalatorvorrichtung nach einem der Ansprüche 1 bis 8, **dadurch gekennzeichnet, dass** das gemeinsame Dosisbett (20) als Blisterverpackung ausgebildet ist, wobei das gemeinsame Dosisbett (20) ausgelegt ist, getrennte Depots der Substanzen (1, 2), welche die vorab abgemessene kombinierte Dosis (100) bilden, aufzunehmen.

10. Trockenpulver-Inhalatorvorrichtung nach einem der Ansprüche 1 bis 9, **dadurch gekennzeichnet, dass** eine biologisch akzeptable, inaktive Substanz (3) zwischen den Depots (1, 2) der zumindest zwei Substanzen deponiert wird, um zu verhindern, dass die zumindest zwei Substanzen in schädlicher Weise aufeinander einwirken, nachdem die kombinierte Dosis (100) gebildet wurde.

## Revendications

1. Inhalateur de poudre sèche, comprenant :
une buse (25) ;
un lit de dose commun (20) ; et
une dose combinée (100) de poudres sèches finement divisées (1, 2) prévues pour être distribuées par l'inhalateur de poudre sèche, la dose combinée (100) comprend des quantités pré-dosées d'au moins deux substances (1, 2), déposées séparément sur le lit de dose commun (20), **caractérisé par** :
un agencement pour fournir un mouvement relatif entre la buse (25) et le lit de dose commun (20), de sorte que la buse (25) passe sur la dose combinée (100) pour une distribution simultanée ou séquentielle des substances (1, 2) au cours d'une simple aspiration d'air et du mouvement relatif.

2. Inhalateur de poudre sèche selon la revendication 1, **caractérisé par** un embout buccal raccordé à la buse (25) et adapté pour recevoir un effort d'aspiration d'air appliqué.

3. Inhalateur de poudre sèche selon la revendication 1 ou 2, **caractérisé en ce que** la buse (25) est initialement positionnée à l'extérieur d'une zone (11, 12) du lit de dose commun (20) sur lequel les substances (1, 2) sont déposées.

4. Inhalateur de poudre sèche selon l'une quelconque des revendications 1 à 3, **caractérisé en ce que** l'agencement est agencé pour fournir le mouvement relatif jusqu'à ce que un courant d'air (26) induit dans la buse (25) dépasse une vitesse d'écoulement de seuil.

5. Inhalateur de poudre sèche selon l'une quelconque des revendications 1 à 4, **caractérisé en ce que** le lit de dose commun (20) est hermétiquement fermé par au moins une feuille (13) et l'inhalateur de poudre sèche comprend en outre un agencement pour ouvrir le joint d'étanchéité permettant à la buse (25) d'avoir accès au lit de dose commun.

6. Inhalateur de poudre sèche selon la revendication 5, **caractérisé en ce que** le lit de dose commun (20) comprend au moins deux compartiments (21, 22), les au moins deux substances (1, 2) sont déposées dans des compartiments séparés (21, 22) et chaque compartiment (21, 22) est prévu avec un joint d'étanchéité séparé (13) pour empêcher l'interaction entre les substances (1, 2) déposées séparément.

7. Inhalateur de poudre sèche selon l'une quelconque des revendications 1 à 6, **caractérisé en ce qu'**au moins l'une des au moins deux substances (1, 2) est composée d'un agent pur, chimique ou biologique.

8. Inhalateur de poudre sèche selon l'une quelconque des revendications 1 à 7, **caractérisé en ce qu'**au moins l'une des au moins deux substances (1, 2) se présente sous la forme d'un composé de poudre sèche se composant d'une quantité effective d'un agent pur, chimique ou biologique mélangé avec des excipients (3) appropriés.

9. Inhalateur de poudre sèche selon l'une quelconque des revendications 1 à 8, **caractérisé en ce que** le lit de dose commun (20) est formé comme un emballage blister, où le lit de dose commun (20) est conçu pour accepter des dépôts séparés des substances (1, 2) composant la dose combinée pré-dosée (100).

10. Inhalateur de poudre sèche selon l'une quelconque des revendications 1 à 9, **caractérisé en ce qu'**une substance inerte (3) biologiquement acceptable est déposée entre les dépôts (1, 2) des au moins deux substances pour empêcher les au moins deux substances d'interagir de manière préjudiciable après la formation de la dose combinée (100).
